# EUROPEAN PATENT SPECIFICATION

(11) **EP 1 492 503 B1**
(45) Date of publication and mention of the grant of the patent: **12.11.2008**
(21) Application number: 03730170.2
(22) Date of filing: 02.04.2003
(51) Int. Cl.: A61K 9/14, A61K 31/352

(54) **STABILIZED NATURAL CANNABINOID FORMULATION**
STABILISIERTE NATÜRLICHE CANNABINOID FORMULIERUNG
FORMULATION DE CANNABINOIDE NATUREL STABILISEE

(30) Priority: 03.04.2002 US 369613 P; 04.04.2002 EP 02076339
(43) Date of publication of application: 05.01.2005
(73) Proprietor: Solvay Pharmaceuticals B.V., 1381 CP Weesp (NL)
(72) Inventor: EISSENS, Anko, C., NL-1381 CP Weesp (NL); VAN DROOGE, Dirk, J., NL-1381 CP Weesp (NL); HINRICHS, Wouter, L.J., NL-1381 CP Weesp (NL); FRIJLINK, Henderik, W., NL-1381 CP Weesp (NL)
(74) Representative: Verhage, Marinus
(86) International application number: PCT/EP2003/050087
(87) International publication number: WO 2003/082246

(56) References cited:
- WO-A-00/78817
- WO-A-91/18091
- WO-A-97/36577
- WO-A-99/32107

## Description

The present invention is related to a pharmaceutical formulation stabilizing natural cannabinoid compounds, especially Δ⁹-tetrahydrocannabinol (THC). The invention further relates to a method for preparing said formulations.

Natural cannabinoid compounds, which can be obtained from several natural sources, but that are normally obtained from *Cannabis Sativa*, can be used as a therapeutic agent for the treatment of a large variety of diseases. For an overview of natural cannabinoid compounds see David T. Brown ed., Cannabis, Harwood Academic Publishers 1998, ISBN 90-5702-291-5. An example of a natural cannabinoid compound is THC, which is on the market as Marinol® (generic name dronabinol). Currently, THC is formulated as a soft gelatin capsule for oral administration in which the drug is dissolved in an oil. The disadvantage is that in this formulation THC is not stable. As a consequence, it has to be stored at low temperatures (4°C). It is clear that a low stability of a compound and the need to store the pharmaceutical formulation in the refrigerator is a serious drawback for a pharmaceutical product.

It is the object of the present invention to provide a formulation for unstable natural cannabinoid compounds like THC that improves the stability of compounds in such a way that they can be stored at ambient conditions for prolonged times. It is a further object to provide a method to obtain the drug substance in a dry powder state. The dry state offers the possibility to develop other dosage forms e.g. dry powder formulations for pulmonary delivery and tablets for oral or sublingual administration.

WO9932107 discloses the use of cyclodextrins for solubilization of THC in a biphasic delivery system or a microsphere delivery system. The solubilising action of cyclodextrins is caused by the formation of so-called inclusion complexes or guest-host complexes. The object of the subject matter of WO9932107 is the solubilization of THC in order to promote absorption from the nasal cavity. Nothing is disclosed in the application about stability of the formulated THC. On beforehand nothing can be concluded about the stabilizing effect of the formation of the guest-host complex as it is known to the person skilled in the art that these complexes sometimes have a stabilizing effect but in other cases lead to deterioration of the active compound due to catalytic effects. Further cyclodextrins have the draw-back of causing mucosal irritation when applied as a nasal or pulmonary formulation. Especially cyclodextrin derivatives which have surfactant properties are irritant for mucosal tissues.

WO9736577 describes the use of dry solid lipid compositions useful for the oral delivery of lipohilic compounds such as natural cannabinoids, said solid lipid composition comprising, apart from the active substance, a solid fat and a phospholipid. The aim of this composition is the enhancement of oral bioavailability and not the enhancement of the stability of the active substance.

WO0078817 discloses the stabilization of alkaline phosphatase by drying the protein from a pure aqueous solution in the presence of inulin, an oligosaccharide. During drying, the protein is encapsulated monomolecularly by a matrix consisting of amorphous inulin, which is in a glassy state. Amongst other things stabilization is achieved because the protein is vitrified and is shielded from its environment. Alkaline phosphatase is, however, a hydrophilic compound which is very soluble in water and it can be formulated directly from an aqueous solution. Further the stabilization relates especially to the preservation of the tertairy and quaternary structure of the protein, which is important for the enzymatic activity.

WO 9118091 describes the use of non-reducing sugar molecules, especially monoglycosides like maltitol, lactitol and palatinit for the preservation of stability of enzymes, such as restriction endonuclease Pst I, and antibodies, which are hydrophilic compounds. According to this patent application stabilized enzymes can be prepared by mixing of the enzyme with the sugar and a proprietary buffer, followed by air drying. This method cannot be used for lipophilic compounds, as these compounds cannot be solved in sufficient amounts in a polar system. Maltitol and lactitol have glass transition temperatures of 44°C (Y. Roos, Carbohydrate Research 1993, 238, 39-48) resp. 33°C at dry conditions.

It has now surprisingly been found that also highly lipophilic compounds like natural cannabinoid compounds can be stabilized against oxidation and isomerization by the incorporation in sugar glasses or sugar alcohol glasses by the mechanism mentioned above. Furthermore it was found that the sugar glass technology also leads to an improved bioavailability. As the natural cannabinoid compounds are incorporated monomolecularly, the dissolution rate of these compounds will be determined by the dissolution rate of the sugar glass. Because the dissolution rate of the sugar glass is much higher than that of the natural cannabinoid compound, the drug will be presented to the absorbing membrane more rapidly.

In a first embodiment the present invention is related to a method of preparation of a pharmaceutical composition comprising a natural cannabinoid compound and a glass of a sugar or a mixture of sugars wherein the natural cannabinoid compound is incorporated in the sugar glass as a monomolecular encapsulation without formation of a guest-host complex, characterized in that
a) said natural cannabinoid compound is dissolved in an organic solvent that is soluble in water and said sugar or mixture of sugars is dissolved in water;
b) the dissolved cannabonoid compound and the dissolved sugar or mixture of sugars are mixed in such a way that a sufficiently stable mixture is obtained;
c) said mixture is freeze dried, spray dried, vacuum dried, or super critical dried.

The invention is also related to a pharmaceutical composition obtainable by the above method, comprising a natural cannabinoid compound and a glass of a sugar or sugar alcohol or a mixture of sugars or sugar alcohols, characterized in that the natural cannabinoid compound is incorporated in the sugar as a monomolecular encapsulation without formation of a guest-host complex. The compound is incorporated in the sugar glass when there is a monomolecular inclusion of substantially every cannabinoid molecule in the sugar matrix. Therefore the formed delivery system according to this embodiment of the invention can be regarded as a monophasic delivery system. The natural cannabinoid molecules are randomly orientated within the sugar glass. In contrast to guest-host complexes like complexes with cyclodextrins, once dissolved there remains no interaction between the cannabinoid compounds and the dissolved sugar molecules.
Incorporation of a cannabinoid compound in the sugar glass will result in a decrease of the glass transition temperature (Tg) of the sugar glass, a disappearance of the Tg of the cannabinoid compound, and a increased dissolution rate of the cannabinoid compound. Furthermore, scanning electron microscopy can indicate whether the compound in incorporated. The most preferred natural cannabinoid compound is THC.
In order to obtain the highest stability the sugar glass preferably has a glass transition temperature (Tg) of above 50°C at the normal environmental conditions and has a low tendency to crystallize. Normal environmental conditions are defined as 20 to 25°C and up to 40% relative humidity.
In the framework of the present invention the expression "natural cannabinoid compound" includes non-natural derivatives of cannabinoids which can be obtained by derivatization of natural cannabinoids and which are unstable like natural cannabinoids.
In the framework of the present invention the expression sugar includes polysugars and the expression sugar alcohols includes poly sugar alcohols. Preferred sugars in the present invention are non-reducing sugars. A non reducing sugar is a sugar, which does not have or can not form reactive aldehyde or ketone groups. Examples of non-reducing sugars are trehalose and fructanes such as inulines.

Prefered non-reducing sugars to use in the present invention are fructans or mixtures of fructans. A fructan is understood to mean any oligo- or polysaccharide which contains a plurality of anhydrofructan units. The fructans can have a polydisperse chain length distribution, and can have a straight or branched chain. Preferably the fructans contain mainly β-1,2 bonds, as in inulin, but they can also contain β-2,6 bonds, as in levan. Suitable fructans can originate directly from a natural source, but may also have undergone modification. Examples of modifications are reactions known per se that lead to a lengthening or shortening of the chain length. In addition to naturally occurring polysaccharides, also industrially prepared polysaccharides, such as hydrolysis products which have shortened chains and fractionated products having a modified chain length are suitable in the present invention. A hydrolysis reaction to obtain a fructan having a reduced chain length can be carried out enzymatically (for instance with endoinulase), chemically (for instance with aqueous acid, physically (for instance thermally) or by the use of heterogeneous catalysis (for instance with an acid ion exchanger). Fractionation of fructans, such as inulin, can be achieved inter alia through crystallization at low temperature, separation with column chromatography, membrane filtration and selective precipitation with an alcohol. Other fructans, such as long-chain fructans, can be obtained, for instance through crystallization, from fructans from which mono-and disaccharides have been removed. Fructans whose chain length has been enzymatically extended can also serve as fructan in the present invention. Further, reduced fructans can be used, which are fructans whose reducing end groups, normally fructose groups, have been reduced, for instance with sodium borohydride, or hydrogen in the presence of a transition metal catalysts. Fructans which have been chemically modified, such as crosslinked fructans and hydroxyalkylated fructans, can also be used. The average chain length in all these fructans is expressed as the number-average degree of polymerization (DP). The abbreviation DP is defined as the average number of sugar units in the oligo- or polymer.
Even more preferred reducing sugars in the present invention are inulins or mixtures of inulins. Inulins are oligo- and polysaccharides, consisting of β-1,2 bound fructose units with an α-D-glucopyranose unit at the reducing end of the molecule and are available with different degrees of polymerization (DP). The preferred inulins are inulins with a DP of greater than 6 or a mixtures of inulins wherein each inulin has a DP of greater than 6. Even more preferred are inulins or mixtures of inulins with a DP of between 10 and 30. Most preferred are inulins or mixtures of inulins with a DP of between 15 and 25. Inulin occurs inter alia in the roots and tubers of plants of the *Liliaceae* and *Compositae* families. The most important sources for the production of inulin are the Jerusalem artichoke, the dahlia and the chicory root. Industrial production starts mainly from the chicory root. The main difference between inulins originating from the different natural sources resides in the degree of polymerization (DP), which can vary from about 6 in Jerusalem artichokes to 10-14 in chicory roots and higher than 20 in the dahlia. Inulin is an oligo- or polysaccharide which in amorphous condition has favorable physicochemical properties for the application as auxiliary substance in pharmaceutical formulations. These physicochemical properties are: (adjustable) high glass transition temperature, no reducing aldehyde groups and normally a low rate of crystallization. Further inulin is non toxic and inexpensive.

The weight ratio of natural cannabinoid compound to sugar or sugar alcohol is typically in the range of between 1:5 to 1:100, more preferably in the range of between 1:10 and 1:50 and most preferred in the range between 1:12 and 1: 25.

The pharmaceutical composition according to the present invention may be further processed into a tablet such as a normal oral tablet, a sublingual tablet, a buccal tablet or an orally disintegrating or dissolving tablet, a capsule, a lozenge, an enema, a suppository, a product for transdermal administration, a powder for pulmonary administration, or a rod or suspension for subcutaneous or intramuscular administration. These forms of administration are known in the art and the person skilled in the art will be capable to process the composition according to the present invention into the desired form of administration. Preferred formulations are those intended for oral administration or pulmonary administration.

An appropriate technique for the preparation of sugar glasses according to the present invention is freeze drying. Also other drying techniques such as spray drying, vacuum drying, and super critical drying can be employed. The first step to prepare sugar glasses with incorporated natural cannabinoid compounds by means of these techniques is to make a solution in which both substances are dissolved. However, due to the hydrophilic nature of sugars and the lipophilic nature of the natural cannabinoid compounds, these compounds are hard to dissolve in the same solvent. It has now been found that this problem can be solved by the application of mixtures of solvents. Water is a good solvent for sugars and sugar alcohols, whereas various organic solvents such as alcohols are good solvents for natural cannabinoid compounds. Since water and alcohols mix very well it is likely that at a certain water/alcohol ratio both substances will dissolve to a certain extent.

Organic solvents which are suitable to form a stable mixture with the sugar, water and the natural cannabinoid compound are solvents which are mixable with water such as dimethylsulfoxide (DMSO), N,N-dimethylformamide (DMF), acetonitrile, ethylacetate and lower alcohols. As the solvents have to be removed by spray drying or freeze drying the solvents should preferably also have a reasonable vapor pressure at the drying temperature. Therefore lower alcohols, defined as C₁-C₆ alcohols, wherein the alkyl chain may be branched or unbranched are preferred. The more preferred alcohols are C₂-C₄ alcohols such as ethanol, n-propyl alcohol and *t-*butyl alcohol. The most preferred solvent is *t*-butyl alcohol.

The ratios between the cannabinoid compound, the solvent, water and the sugar or mixture of sugars should be chosen in such a way that a sufficiently stable solution is obtained. Optionally a surfactant can be added to improve the stability. A solution is judged as sufficiently stable if no clouding appears in the solution within the time of processing e.g. within 120 minutes, 60 minutes, 30 minutes or 10 minutes. For a spray drying process a typical time of processing is 30 minutes. For a freeze drying process the solution should be clear until it is frozen. A typical time of processing here is 10 minutes.
The amount of water after the drying process preferably is below 3%. The amount of solvent is preferably below 3%. It will be clear for a person skilled in the art that the time required for drying can be derived from parameters like sample thickness, sample temperature, pressure, and condenser temperature.

Although the use of a spray drying process for the preparation of sugar glasses of cannabinoid compounds leads to a significant improvement of the stability of the compounds, the best results are obtained with a freeze drying process. Therefore the most preferred method of drying in the present invention is freeze drying.
In the first phase of the freeze drying process the solution is frozen. This first phase should preferably be performed rapidly and should reduce the sample temperature to below Tg', which is the temperature of the freeze concentrated fraction (see D.L. Teagarden, Eur. J. Pharm. Sci., 15, 115-133, 2002). Freeze drying below the Tg' results in a porous cake, while a collapsed cake is obtained above the Tg'. A porous cake is preferred because it can be processed more easily into e.g. a powder for tabletting or formulations for pulmonary delivery. Moreover, freeze drying above the Tg' may lead to crystallization of the sugar. This will prevent the incorporation of the drug in a glass and as a result reduced stabilization will be achieved.

The following example is only intended to further illustrate the invention, in more detail.

### Example 1. Preparation and properties of inulin glasses of Δ⁹-tetrahydrocannabinol.

### Materials

Inulin, type TEX!803, was provided by Sensus, Roosendaal, The Netherlands. Purified Δ⁹-tetrahydrocannabinol (THC) was a gift of Unimed. All other chemicals were of reagent or analytical grade and purchased from commercial suppliers.

### Methods

### Physico-chemical characterization of inulin

### Determination of the degree of polymerisation of inulin

The average degree of polymerisation (DP) of inulin was determined as follows: an inulin solution was acidified to a pH of 1.45 by adding 3 N HCl. Subsequently, the temperature was raised to 80°C by which the inulin was degraded to fructose and glucose. After cooling to room temperature, the pH was adjusted to 6-8 by adding 1.5 M NaOH. The fructose/glucose ratio was determined by means of HPLC. An Aminex HPX-87C column was used. Samples were eluated with MilliQ-water of 80°C at a flow rate of 0.6mUmin. An IR detector was used to measure the amounts of fructose and glucose. The DP is the ratio of the fructose content and the glucose content plus one.

### Determination of the number of reducing groups

The number of reducing groups was determined by means of the Sumner-assay according to the following procedure. A solution of 20 g NaK-tartrate tetrahydrate, 1 g dinitrosalicylic acid,1 g NaOH, and 200 mg phenol in 100 mL water was prepared. To 1.5 ml of this solution, 1.0 mL of an aqueous solution containing the sugar to be analysed was added. Subsequently, 100 µL of a freshly prepared solution of 0.24 M of Na₂SO₃ in water was added to this mixture. The resulting mixture was vortexed and then placed in a waterbath of 95°C. After 15 min, the samples were removed from the waterbath and allowed to cool to room temperature. The extinction of the samples was measured at 620 nm. The calibration curve was made using aqueous solutions with a glucose concentration of 0.10-1.00 mg/mL. Measurements were performed in triplicate.

### Differential Scanning Calorimetry (DSC)

The glass transition temperature (Tg) of freeze dried inulin equilibrated at 0%, 45% and 60% RH was determined by modulated DSC (DSC 2920 differential scanning calorimeter, TA instruments, Gent, Belgium). A modulation amplitude of ± 0.318°C every 60 sec and a heating rate of 2°C/min was used. During measurement, the sample cell was purged with nitrogen at a flow rate of 35 mL/min. The midpoint of the deflection in the reversing heat flow versus temperature curve was taken as the Tg. The Tg was determined in duplicate.
The glass transition temperature of the freeze concentrated fraction (Tg') of a 9.6 % w/v solution of inulin in 60/40 v/v water/t-butyl alcohol mixture was measured by means of conventional DSC. Solutions were cooled to -70°C with a cooling rate of 10°C/min. Subsequently, the samples were heated to 40°C with a rate of 2°C/min. During these measurements, the sample cell was purged with helium at a flow rate of 35 mL/min. The midpoint of the deflection in the heat flow versus temperature curve was taken as the Tg'. The Tg' was determined in duplicate.

### Physical stability amorphous inulin

To evaluate the physical stability of amorphous inulin, porous cakes of amorphous inulin obtained by means of freeze drying were humidified at 20°C by transferring them into climate chambers conditioned at 45% or 60% RH respectively. After equilibration, the samples were judged visually whether they remained unchanged or were collapsed.

### Dynamic vapour sorption

Water sorption isotherm of freeze dried inulin was measured at ambient pressures and 25°C using a gravimetric sorption analyser (DVS-1000 Water Sorption Instrument, Surface Measurement Systems Limited, London, UK). The uptake of water by inulin was measured from 0% to 90% RH with steps of 10% RH. The initial sample weight was about 10 mg. It was assumed that equilibrium was reached when the change of weight was less than 0.9 µg during a ten minutes period.

### Physico-chemical characterization of THC

### Solubility in water

Pure water was added to an excess of THC. The resulting dispersion was stirred at 20°C using a magnetic stirrer. After 3 days the dispersion was centrifuged and the concentration of THC in the supernatant was determined spectrophotometrically at a wavelength of 210 nm. The sample was diluted with ethanol. A calibration curve was established using solutions of THC in ethanol of known concentrations (1.244-12.44 µg/mL).

### Dynamic vapour sorption

The water sorption of THC was determined according to the procedure described above for inulin. The THC was dissolved in methanol before it was put in the DVS-1000 instrument. During initial exposure to a dry nitrogen flow, the methanol was evaporated. As soon as about 90% of the solvent was evaporated, additional THC solution was added to the sample-cup. This procedure was repeated until 15 mg of pure THC was present in the sample cup. After the evaporation of the last methanol, the relative humidity was increased from 0% to 90% in steps of 10%.

### Differential Scanning Calorimetry (DSC)

The thermal behavior of THC was determined by mDSC. A modulation amplitude of ± 0.318°C every 60 sec and a heating rate of 2°C/min was used. During measurement, the sample cell was purged with nitrogen at a flow rate of 35 mL/min. A blob of pure THC was put in the sample cup. After initial cooling the sample was first scanned till 50°C. In this way the blob was able to spread over the entire bottom of the sample pan, thereby increasing the surface available for heat transfer during the second scan. The sample was then cooled until -40°C and heated to 350°C.

### Production of THC containing samples

### Preparation of solutions for spray drying or freeze drying

Three different formulations were prepared for spray drying and one for freeze drying (Table 2). Formulations 5, 6, 9 and 12 were prepared by dissolving, separately inulin in water and THC in the appropriate alcohol.
The suitable volume ratio of water/alcohol was investigated by testing the stability of 10% w/v inulin solutions with different ratio's water/alcohol. Inulin was dissolved in different amounts of water (3 to 7 mL). Subsequently different amounts of alcohol were added until a total volume of 10 mL. For THC the same procedure was followed, but now water was added to the alcoholic THC solution. The solution was judged as sufficiently stable if no clouding appeared within the time of processing. For spray drying batches were made which required up to half an hour of spraying. Therefore the solution should be clear for at least that period of time. For freeze drying the solution should be clear until it is frozen. In this case ten minutes is sufficient. Furthermore, it was investigated whether the aqueous inulin solution could be added slowly or should be mixed instantaneously.

**Table 2: Formulations for spray drying and freeze drying**

| **Formulation** | **Drying method** | **Solvent** | **[Inulin]** **(mg/mL)** | **THC/inulin** **(m%)** |
|---|---|---|---|---|
| 9 | Spray drying | H₂O/EtOH = 50/50(v/v) | 47.73 | 4.00% |
| 5 | Spray drying | H₂O/1-PrOH = 60/40(v/v) | 49.00 | 3.34% |
| 6 | Spray drying | H₂O/1-PrOH = 60/40(v/v) | 46.17 | 7.77% |
| 12 | Freeze drying | H₂Ot-BuOH = 60/40(v/v) | 96.00 | 4.00% |

### Spray drying

Spray drying was performed using a Büchi 190 mini spray dryer (Büchi, Flawil, Switzerland). Typical operating conditions were according to the following settings: nitrogen-gas inlet temperature: 148°C which gave an outlet temperature of 87°C, drying air flow 525 Uh, aspirator flow setting: 20, and pump control setting: 6. After spray drying, the formed powder was collected in a 50 mL bottle and flushed with nitrogen for about 15 minutes. The product was stored at -18 °C.

### Freeze drying

Freeze drying was performed using a Christ model Alpha 2-4 lyophilizer (Salm en Kipp, Breukelen, The Netherlands). In a typical experiment, 20 mL glass vials were charged with 2-5 mL solution. The solutions were frozen in liquid nitrogen and subsequently lyophilized at shelf temperature of -30°C, a condenser temperature of -53°C, and a pressure of 0.220 mBar for 1-3 days. Subsequently, the shelf temperature was gradually raised to 20°C and pressure was gradually decreased to 0.05 mBar during 6 hours. The samples were stored in a vacuum desiccator for at least one day.

### Stability study of THC containing samples

Samples were stored under five different conditions; given in Table 3. At different time intervals samples were taken and the amount of nondegraded THC was determined by means of HPLC. Pure THC and a physical mixture of THC and inulin were used as controls. Samples of pure THC were made as follows. 720.5 mg of THC was dissolved in 20.00 mL of methanol. 70 µL of this solution was transferred into a glass vial with a diameter of 24 mm. Subsequently the solvent was allowed to evaporate in a flow of dry nitrogen, leaving 2.52 mg of pure THC in the vial. A physical mixture was prepared by weighing about 192 mg of inulin into a vial with a diameter of 24 mm. Subsequently, 200 µL of a 36.025 mg/mL methanolic solution of THC was added, yielding a mixture containing 4.0% THC by mass.

**Table 3: Storage conditions THC containing samples**

| **Temperature (°C)** | **Relative humidity (%)** | **Atmosphere** |
|---|---|---|
| 20 | 0 | low [O₂] |
| 20 | 45 | air |
| 20 | 60 | air |
| 47 | 0 | low [O₂] |
| 47 | 5 | air |

### THC-analysis

The samples were analysed by means of HPLC. They were prepared as follows. Methanol was added to samples. An ultrasonic treatment of ten minutes dispersed the product throughout the methanol. The suspension thus obtained, was shaken manually. After two days of extraction a sample was taken. The sample was centrifuged and the supernatant was diluted with methanol. In a control experiment, it was shown that ultrasonic treatment induced no degradation of THC. During the two days of extraction, no significant degradation of THC was measured. An ISCO model 2350 system equipped with a Photodiode Array UV-VIS Detector (Shimadzu SPD-M6A model) and a Chrompack Nucleosil 100 C18 column (4.6x250 mm) was used. Samples (20 µL) were injected with a Kontron Instruments HPLC 360 Autosampler and eluted with a mixture of methanol/water = 86/14 (v/v). The flow rate was 1.5 mL/min. The absorbance was measured at 214 nm. The collected data were analysed using SPD-MXA software. In a chromatogram of untreated THC, a large peak was observed at a retention time of 7.5 min. In a chromatogram of THC which was intentionally partially degraded, the peak at a retention time of 7.5 min decreased in size while at shorter retention times new peaks appeared. The peak at a retention time of 7.5 min was ascribed to Δ⁹-THC. The other peaks were ascribed to degradation products. The content of (non-degraded) THC in processed samples was calculated from the area under the peak at an elution time of 7.5 min. A calibration curve was established using solutions of THC in methanol of known concentrations (0-122 µg/mL). In every HPLC-run some calibration points were included. The solutions used for this purpose showed no significant degradation during a period of 2 weeks at 4°C. Measurements were performed at least in duplicate.

### RESULTS

### Physico-chemical characterization of inulin

The physico-chemical characteristics of the inulin used are summarized in Table 4.

**Table 4: Physico-chemical characterization of inulin glasses**

| | |
|---|---|
| Average degree of polymerization | 23 |
| % sugar units containing reducing groups | 5.9 ± 0.1 |
| Tg | 155.4 ± 0.1 °C |
| Tg' | -24°C |
| Physical stability at 20°C | Stable at RH ≤ 45%; collapsed at RH ≥ 60% |
| Hygroscopicity | Change in mass = 0.22 * RH(%) + 0.61 |

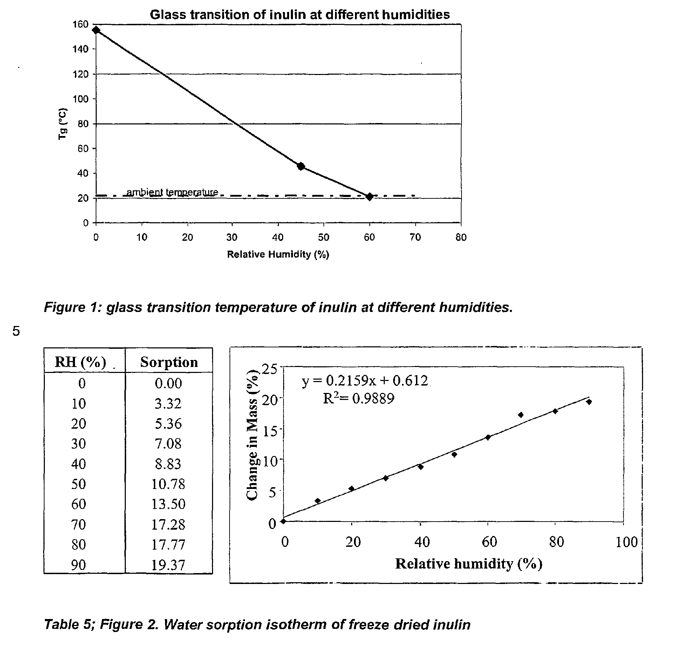

A DP of inulin of 23 was found. For several reasons this value should be regarded as an indication. Inulin consists of linear β-D-(2→1) linked fructose oligomers ending with a α-D-(1→2) glucopyranose ring. Therefore, the DP can be calculated from the glucose/fructose ratio as presented here. However, commercially available inulins may contain inulin species of which the glucose endgroup is cleaved. The presence of these species will cause an overestimation of the DP. On the other hand commercially available inulins may also contain small amounts of glucose. The presence of these species will cause an underestimation of the DP.
Due to the specific linkages between the monosaccharide rings, inulin should contain no reducing groups. However, the Sumner assay showed that 5.9 ± 0.1% of sugar units of the inulin used in this study contained reducing groups. The presence of reducing groups can be predominately ascribed to inulin species of which the glucose endgroup is cleaved although the presence of monosaccharides may have contributed too. These monosaccharides can be glucose and fructose. Fructose is a nonreducing sugar. However, during the Sumner assay, the sugar is subjected to a high temperature by which fructose can be easily converted into glucose (Lobry de Bruyn van Ekenstein rearrangement). Indeed in control experiments, it was found that fructose displayed one reducing group per molecule in the assay (data not shown). Therefore, the measured amount of reducing groups is probably overestimated.
A glass transition temperature (Tg) of inulin of 155.4 ± 0.1°C was found. This value is substantially higher than the Tgs of trehalose (120°C) and sucrose (76°C), sugars which are frequently used to stabilize unstable drugs. A high Tg is important because at temperatures above the Tg the material changes into the rubbery state. In the rubbery state the molecular mobility is strongly increased compared to glassy state, as a consequence the degradation rate of the enclosed drug substance is strongly increased. Besides that, also crystallization can occur in the rubbery state. During crystallization, the incorporated drug substance is expelled from the stabilizing matrix and the protection is completely lost. The Tgs may seem very high. However, sugar glasses absorb water upon exposure to humidified air (see below). Water acts as a plasticizer for sugar glasses and strongly decreases the Tg. Therefore, inulin glasses can absorb much more water than trehalose or sucrose glasses before the Tg is decreased to room temperature.
A Tg' of inulin of -24°C was found. Also this value is higher than the Tg's of trehalose (-36°C) and sucrose (-39°C). When freeze drying is chosen as the method of drying, it is preferrable that Tg' is relatively high, because the sample temperature should remain below the Tg'. When the sample temperature is above Tg', the freeze concentrated fraction is in the rubbery state and as mentioned above the molecular mobility is relatively high. Because concentration of the drug substance in the freeze concentrated fraction is very high, the degradation rate can be increased when compared to the starting solution. Furthermore, also in this case crystallization of the sugar may easily occur with concomitant deteriorating effects to the drug substance. Furthermore freeze drying below the Tg' results in a porous cake, while a collapsed cake is obtained above the Tg'. A porous cake is preferred because it can be processed more easily into e.g. a powder for tabletting or formulations for pulmonary delivery.
The physical stability of inulin glass at 20°C was evaluated by exposing the glass to air of various relative humidities. It was found that porous cakes of inulin prepared by freeze drying remained unaffected up to an RH of 45%. At an RH of 60%, however, the porous cake collapsed. This means that at an RH between 45% and 60%, the sample absorbed water to such an extent that the Tg is passed. A short period of exposure to 60% RH may be applied to the freeze dried cake to have it partially collapsed. This partially collapsed material may form a suitable fast dissolving tablet with sufficient strength. The Tgs of freeze dried inulin after equilibration in 0, 45% and 60% RH are depicted in figure 1.

The moisture uptake of freeze dried inulin exposed to air of relative humidities ranging from 0 to 90% at 25°C was measured using a gravimetric sorption analyser. Over the whole range of relative humidities, a linear relationship was found between the water uptake and the RH to which the sample was exposed (Table 5; Figure 2). As found above, the Tg is passed at an RH between 45% and 60%. The linear relationship indicates that during the time frame of the experiment (hours) no crystallization of inulin takes place. When crystallization takes place and anhydrous crystals are formed the water content of the sample will drop to close to zero. On the other hand when crystals are formed which enclose water molecules, the water content of the sample remains more or less the same with increasing RH. These phenomena were observed with water sorption experiments with amorphous sugars like trehalose, sucrose, and lactose. Therefore, the results indicate that amorphous inulin crystallizes less easily than amorphous trehalose, sucrose, and lactose.

### Physico-chemical characterisation of THC

### Solubility

The solubility of THC was found to be below 1 µg/mL (approximately 0.5 µg/mL).

### Dynamic vapour sorption.

Pure THC was found to absorb only 0.3% water after exposure to 90% RH. This extent of water uptake can probably be ascribed to adsorption onto rather than absorption into THC.

### Differential Scanning Calorimetry

In the thermogram of THC a Tg of 10°C was found. Furthermore an endothermic peak with an onset at 200°C was found. From a thermodynamic point of view, it is expected that just above the Tg crystallization takes place. However, it is known that THC does not crystallize easily. As a consequence, at ambient temperature, THC is in the rubbery or liquid state. The endothermic peak is due to evaporation.

### Production of THC containing Samples

### Water-alkanol solutions for spray drying or freeze drying

The three relevant alcohol's were added to a solution of inulin in water. It was determined for how long the obtained solution stayed clear. After 1 g of inulin was dissolved in 4 mL water, water and/or alcohol was added to a total volume of 10 mL, yielding a 10% w/v solution. The largest concentration of alcohol was thus obtained. THC was dissolved in the alcohol of interest. Subsequently, alcohol and/or water was added to give 0.4% w/v solutions. The compositions required to obtain a stable solution (defined in Materials and Methods) are given in table 5.

**Table 5: Ratio's water-alcohol in solutions of inulin and THC.**

| | | |
|---|---|---|
| | %v/v water | alcohol |
| THC | 53% maximum | EtOH (ethanol) |
| | 62% maximum | n-PrOH (n-propanol) |
| | 63% maximum | TBA (t-butanol) |
| Inulin | 50% minimum | EtOH |
| | 60% minimum | n-PrOH |
| | 60% minimum | TBA |

Solutions for spray drying were prepared by adding the aqueous inulin solution to the THC solution. It turned out that this must be done quite fast, to prevent the inulin from clouding the mixture. The solutions stayed clear during the time necessary to spray the solution. The THC solution to be freeze dried was prepared by dissolving 690 mg THC in 20 mL TBA. Glass vials of 20 mL were each filled with 0.23 mL of the THC solution. Subsequently, the solutions were diluted with 0.57 mL of pure TBA. After that, 1.2 mL of an aqueous inulin solution (160 mg/mL) was added, the vials were shaken manually and frozen immediately afterwards.

### Recovery of THC after drying

The amount of THC in the spray dried samples, immediately after production, was lower than expected. Initially recoveries of about 50% were found. After changing both the atomizing gas flow and the gas flow from the heater to nitrogen, the recovery increased to 75%. In case of freeze drying, 100% of the expected amount of THC was found in the samples after the drying procedure.

### Characterisation of THC containing samples

### Scanning Electron Microscopy

Scanning electron microscopy (SEM) photo's of the spray dried products showed the existence of agglomerates of small particles. These particles, having diameters of 1 to 5 µm, were hollow. The small size and the decreased density of the spray dried particles make them excellent for processing into dry powder formulations for inhalation. A SEM photo of a reference product (inulin without THC which was spray dried under the same conditions and with the same solvents) showed no differences. No THC spots are noticed on the particle surfaces of THC containing samples, indicating that THC is incorporated in the inulin matrix.

### Stability of THC containing samples

The samples were exposed to conditions with O₂ or at low O₂ (indicated as nitrogen in the figures), at 20°C and 47°C respectively. Furthermore, they were exposed to two different humidities at 20°C, as summarized before. The spray dried products showed a slight change in colour after they were collected from the spray-dryer.

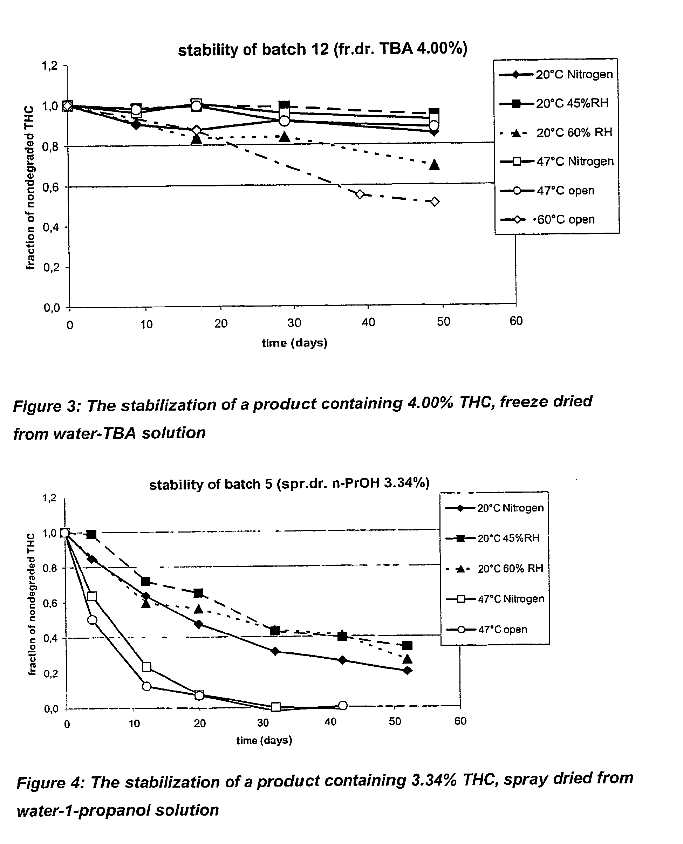

Figures 3-6 show the results of the batches 12, 5, 6, and 9. The amount of THC was determined. In the figures the fraction of Δ⁹-THC present in the samples after several exposure times is plotted for the five different climates.
The freeze dried sample (batch 12) is depicted in figure 3. Next to the five climates described before, some samples of this batch were exposed to 60°C 0%RH. Figure 4 shows the stability data of the batch that was spray dried from a solution of 1-PrOH and water, containing 3.34% THC, figure 5 shows a batch with a higher THC content; 7.77% but also spray dried from a water-1-propanol solution. Figure 6, shows the stability data of the batch that was spray dried from a solution of ethanol and water, containing 4.00% THC.

The results from the spray dried batches show that the stability of the THC is improved by the formulation. The temperature has the biggest influence on the degradation rate. Moisture and oxygen are of less importance. However, it should be noticed that the samples stored under nitrogen were probably contaminated with oxygen to a certain level.

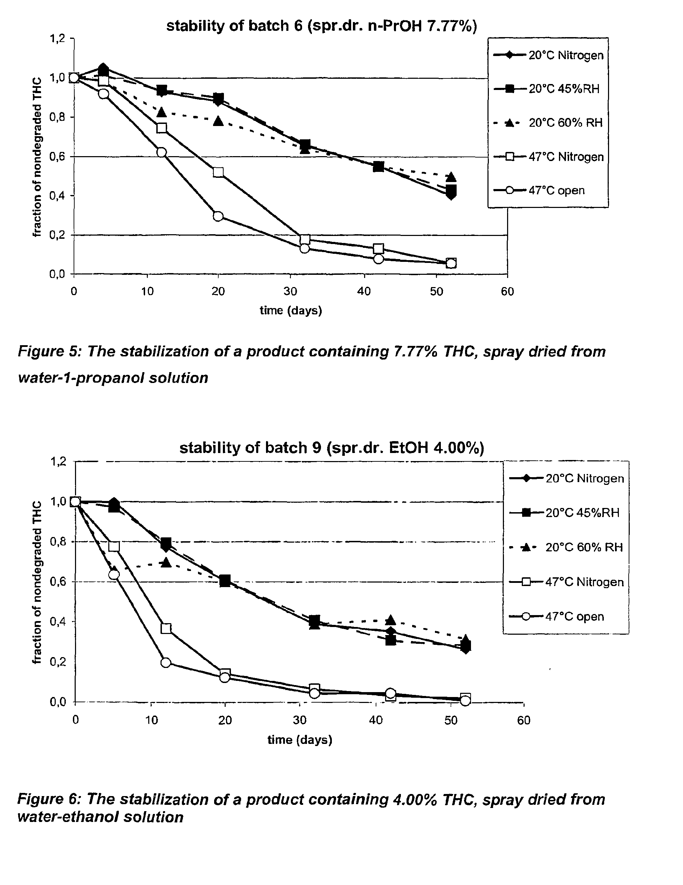

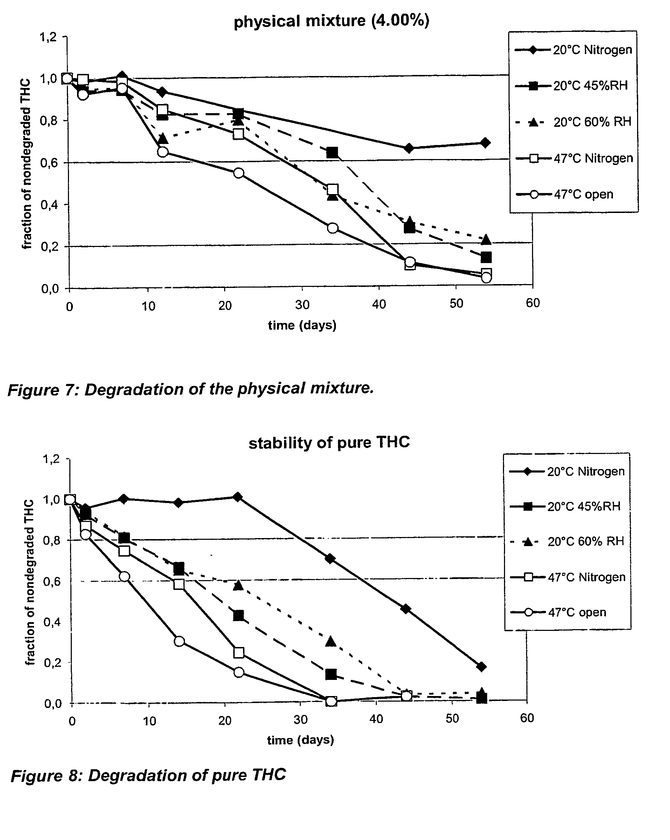

The different figures clearly show that the stability of the freeze dried product was superior, when compared with the physical mixture and the pure THC (see figures 7 and 8). Apparently, the process by which the sugar glasses are prepared strongly influences the stability of the product.
As can be seen in figure 5 the degradation in the freeze dried product is minimal for all tested conditions, except for 60% RH. However the somewhat lower concentration found here might also be caused by the fact that at this condition the material is collapsed which makes the extraction procedure less effective.

### Reference batches

To test the stabilizing capacity of inulin, the data shown above should be compared with a batch with the same chemical and physical structure, but without the inulin. This would imply that a reference batch consists of separate inulin molecules, in fact a vapour of THC. Because this is impractical, two other reference-batches are prepared: a physical mixture containing about 4% THC and 96% unprocessed inulin and pure THC. The results are presented in figures 7 and 8 respectively.

It has to be mentioned that during the preparation of the physical mixture the solution of THC in methanol softened the inulin powder to some extend. After evaporation of the methanol, a more or less solid film of inulin and THC appeared at the bottom of the vial. The low porosity film causes an extra protection of this reference material. Besides that, it is possible that the mixing of the methanolic THC solution with the sugar already results in inclusion of a part of the THC.
It should be emphasized that the self protection is also relevant in the pure THC samples since they form also a shielding film.

## Claims

1. A method of preparation of a pharmaceutical composition comprising a natural cannabinoid compound and a glass of a sugar, a sugar alcohol, a mixture of sugars or a mixture of sugars alcohols, wherein the natural cannabinoid compound is incorporated in the sugar glass as a monomolecular encapsulation without formation of a guest-host complex, **characterized in that**
a) said natural cannabinoid compound is dissolved in an organic solvent that is soluble in water and said sugar, sugar alcohol, mixture of sugars or mixture of sugar alcohols is dissolved in water;
b) the dissolved cannabonoid compound and the dissolved sugar, sugar alcohol, mixture of sugars or mixture of sugar alcohols are mixed in such a way that a sufficiently stable mixture is obtained;
c) said mixture is freeze dried, spray dried, vacuum dried, or super critical dried.

2. A method according to claim 1, **characterized in that** said sugar or mixture of sugars is a non-reducing sugar or a mixture of non-reducing sugars.

3. A method according to claims 1 and 2, **characterized in that** said natural cannabinoid compound is Δ⁹-tetrahydrocannabinol.

4. A method according to claims 1 - 3, **characterized in that** said sugar glass has a glass transition temperature of above 50°C at normal environmental conditions.

5. A method according to claims 1 -4, **characterized in that** said sugar or mixture of sugars is a fructane or a mixture of fructanes.

6. A method according to claim 5, **characterized in that** said fructane or mixture of fructanes is inulin or a mixture of inulins, preferably inulin with a DP of greater than 6 or a mixtures of inulins wherein each inulin has a DP of greater than 6.

7. A method according to claim 6, **characterized in that** said inulin or each inulin in the mixture has a DP of between 10 and 30, preferably between 15 and 25.

8. A method according to claim 1-7, **characterized in that** said organic solvent is a C₁-C₆ alcohol, preferably a C₂-C₄ alcohol.

9. A method according to claim 8, **characterized in that** said alcohol is selected from the group consisting of ethanol, n-propanol and *t*-butyl alcohol and preferably is *t*-butyl alcohol.

10. A method according to claims 1-9, **characterized in that** said pharmaceutical composition is prepared by freeze drying.

11. A method according to claims 1-10, **characterized in that** said pharmaceutical composition is further processed into a tablet such as a normal oral tablet, a sublingual tablet, a buccal tablet or an orally disintegrating or dissolving tablet, a capsule, a lozenge, an enema, a suppository, a product for transdermal administration, a powder for pulmonary administration, or a rod or suspension for subcutaneous or intramuscular administration.

12. A pharmaceutical composition obtainable by the method as claimed in claims 1-11.

13. A pharmaceutical composition according to claim 12 in the form of a tablet such as a normal oral tablet, a sublingual tablet, a buccal tablet or an orally disintegrating or dissolving tablet, a capsule, a lozenge, an enema, a suppository, a product for transdermal administration, a powder for pulmonary administration, or a rod or suspension for subcutaneous or intramuscular administration.

14. A pharmaceutical composition according to claims 12 and 13 intended for oral administration.

15. A pharmaceutical composition according to claims 12 and 13 intended for pulmonary administration.

## Patentansprüche

1. Verfahren zur Herstellung einer pharmazeutischen Zusammensetzung, umfassend eine natürliche Cannabinoidverbindung und ein Glas aus einem Zucker, einem Zuckeralkohol, einem Gemisch aus Zuckern oder einem Gemisch aus Zuckeralkoholen, worin die natürliche Cannabinoidverbindung in dem Zuckerglas als monomolekulare Einkapselung ohne Bildung eines Gast-Wirt-Komplexes eingeschlossen wird, **dadurch gekennzeichnet, dass**
a) besagte natürliche Cannabinoidverbindung in einem organischen Lösungsmittel gelöst wird, das in Wasser löslich ist, und besagter Zucker, Zuckeralkohol, besagtes Gemisch aus Zuckern oder Gemisch aus Zuckeralkoholen in Wasser gelöst wird;
b) die gelöste Cannabinoidverbindung und der gelöste Zucker, Zuckeralkohol, das gelöste Gemisch aus Zuckern oder Gemisch aus Zuckeralkoholen in solch einer Weise gemischt werden, dass ein ausreichend stabiles Gemisch erhalten wird;
c) besagtes Gemisch gefriergetrocknet, sprühgetrocknet, vakuumgetrocknet oder superkritisch getrocknet wird.

2. Verfahren gemäß Anspruch 1, **dadurch gekennzeichnet, dass** besagter Zucker oder besagtes Gemisch aus Zuckern ein nicht-reduzierender Zucker oder ein Gemisch aus nicht-reduzierenden Zuckern ist.

3. Verfahren gemäß Ansprüchen 1 und 2, **dadurch gekennzeichnet, dass** besagte natürliche Cannabinoidverbindung Δ⁹-Tetrahydrocannabinol ist.

4. Verfahren gemäß Ansprüchen 1-3, **dadurch gekennzeichnet, dass** besagtes Zuckerglas eine Glasübergangstemperatur von über 50°C bei normalen Umgebungsbedingungen hat.

5. Verfahren gemäß Ansprüchen 1-4, **dadurch gekennzeichnet, dass** besagter Zucker oder besagtes Gemisch aus Zuckern ein Fruktan oder ein Gemisch aus Fruktanen ist.

6. Verfahren gemäß Anspruch 5, **dadurch gekennzeichnet, dass** besagtes Fruktan oder Gemisch aus Fruktanen Inulin oder ein Gemisch aus Inulinen ist, vorzugsweise Inulin mit einem DP von größer als 6, oder ein Gemisch aus Inulinen, worin jedes Inulin einen DP von größer als 6 hat.

7. Verfahren gemäß Anspruch 6, **dadurch gekennzeichnet, dass** besagtes Inulin oder jedes Inulin in dem Gemisch einen DP von zwischen 10 und 30, vorzugsweise zwischen 15 und 25 hat.

8. Verfahren gemäß Ansprüchen 1-7, **dadurch gekennzeichnet, dass** besagtes organisches Lösungsmittel ein C₁-C₆-Alkohol, vorzugsweise ein C₂-C₄-Alkohol ist.

9. Verfahren gemäß Anspruch 8, **dadurch gekennzeichnet, dass** besagter Alkohol ausgewählt wird aus der Gruppe bestehend aus Ethanol, n-Propanol und *t*-Butylalkohol und vorzugsweise *t*-Butylalkohol ist.

10. Verfahren gemäß Ansprüchen 1-9, **dadurch gekennzeichnet, dass** besagte pharmazeutische Zusammensetzung durch Gefriertrocknen hergestellt wird.

11. Verfahren gemäß Ansprüchen 1-10, **dadurch gekennzeichnet, dass** besagte pharmazeutische Zusammensetzung weiter in eine Tablette, wie eine normale orale Tablette, eine sublinguale Tablette, eine bukkale Tablette oder eine oral zerfallende oder sich lösende Tablette, eine Kapsel, eine Pastille, ein Klistier, ein Zäpfchen, ein Produkt für transdermale Verabreichung, ein Pulver für pulmonale Verabreichung, oder ein Stäbchen oder eine Suspension für subkutane oder intramuskuläre Verabreichung verarbeitet wird.

12. Pharmazeutische Zusammensetzung, welche durch das Verfahren wie in Ansprüchen 1-11 beansprucht erhältlich ist.

13. Pharmazeutische Zusammensetzung gemäß Anspruch 12 in der Form einer Tablette, wie einer normalen oralen Tablette, einer sublingualen Tablette, einer bukkalen Tablette oder einer oral zerfallenden oder sich lösenden Tablette, einer Kapsel, einer Pastille, eines Klistiers, eines Zäpfchens, eines Produkts für transdermale Verabreichung, eines Pulvers für pulmonale Verabreichung oder eines Stäbchens oder einer Suspension für subkutane oder intramuskuläre Verabreichung.

14. Pharmazeutische Zusammensetzung gemäß Ansprüchen 12 und 13, welche für orale Verabreichung beabsichtigt ist.

15. Pharmazeutische Zusammensetzung gemäß Ansprüchen 12 und 13, welche für pulmonale Verabreichung beabsichtigt ist.

## Revendications

1. Procédé de préparation d'une composition pharmaceutique comprenant un composé cannabinoïde naturel et un verre d'un sucre, d'un alcool de sucre, d'un mélange de sucres ou d'un mélange d'alcools de sucre, où le composé cannabinoïde naturel est incorporé dans le verre de sucre sous forme d'une encapsulation monomoléculaire sans formation d'un complexe d'inclusion, **caractérisé en ce que**
a) ledit composé cannabinoïde naturel est dissous dans un solvant organique qui est soluble dans l'eau et ledit sucre, alcool de sucre, mélange de sucres ou mélange d'alcools de sucre est dissous dans l'eau ;
b) le composé cannabinoïde dissous et le sucre, alcool de sucre, mélange de sucres ou mélange d'alcools de sucre dissous sont mélangés de telle manière qu'un mélange suffisamment stable est obtenu ;
c) ledit mélange est lyophilisé, séché par pulvérisation, séché sous vide ou séché par séchage supercritique.

2. Procédé selon la revendication 1 **caractérisé en ce que** ledit sucre ou mélange de sucres est un sucre non réducteur ou un mélange de sucres non réducteurs.

3. Procédé selon les revendications 1 et 2 **caractérisé en ce que** ledit composé cannabinoïde naturel est le Δ⁹-tétrahydrocannabinol.

4. Procédé selon les revendications 1-3 **caractérisé en ce que** ledit verre de sucre a une température de transition vitreuse supérieure à 50°C dans les conditions environnementales normales.

5. Procédé selon les revendications 1-4 **caractérisé en ce que** ledit sucre ou mélange de sucres est un fructane ou un mélange de fructanes.

6. Procédé selon la revendication 5 **caractérisé en ce que** ledit fructane ou mélange de fructanes est l'inuline ou un mélange d'inulines, de préférence une inuline ayant un DP supérieur à 6 ou un mélange d'inulines où chaque inuline a un DP supérieur à 6.

7. Procédé selon la revendication 6 **caractérisé en ce que** ladite inuline ou chaque inuline dans le mélange a un DP entre 10 et 30, de préférence entre 15 et 25.

8. Procédé selon les revendications 1-7 **caractérisé en ce que** ledit solvant organique est un C₁-C₆ alcool, de préférence un C₂-C₄ alcool.

9. Procédé selon la revendication 8 **caractérisé en ce que** ledit alcool est choisi dans le groupe consistant en l'éthanol, le n-propanol et l'alcool *t*-butylique et est de préférence l'alcool *t*-butylique.

10. Procédé selon les revendications 1-9, **caractérisé en ce que** ladite composition pharmaceutique est préparée par lyophilisation.

11. Procédé selon les revendications 1-10, **caractérisé en ce que** ladite composition pharmaceutique est transformée encore en un comprimé comme un comprimé oral normal, un comprimé sublingual, un comprimé gingivo-jugal ou un comprimé à désintégration ou dissolution orale, une capsule, une pastille, un lavement, un suppositoire, un produit pour l'administration transdermique, une poudre pour l'administration pulmonaire, ou un bâton ou suspension pour l'administration sous-cutanée ou intramusculaire.

12. Composition pharmaceutique pouvant être obtenue par le procédé selon les revendications 1-11.

13. Composition pharmaceutique selon la revendication 12 sous forme d'un comprimé comme un comprimé oral normal, un comprimé sublingual, un comprimé gingivo-jugal ou un comprimé à désintégration ou dissolution orale, une capsule, une pastille, un lavement, un suppositoire, un produit pour l'administration transdermique, une poudre pour l'administration pulmonaire, ou un bâton ou suspension pour l'administration sous-cutanée ou intramusculaire.

14. Composition pharmaceutique selon les revendications 12 et 13 destinée à l'administration orale.

15. Composition pharmaceutique selon les revendications 12 et 13 destinée à l'administration pulmonaire.
